(19) Europäisches Patentamt
European Patent Office
Office européen des brevets

(11) Veröffentlichungsnummer: **0 076 379**
**B1**

(12) **EUROPÄISCHE PATENTSCHRIFT**

(45) Veröffentlichungstag der Patentschrift: **15.05.85**

(51) Int. Cl.[4]: **C 07 C 69/72**, C 07 C 67/31

(21) Anmeldenummer: **82107753.4**

(22) Anmeldetag: **24.08.82**

(54) **Verfahren zur Herstellung von 4-Alkoxyacetessigestern.**

(30) Priorität: **01.10.81 CH 6321/81**

(43) Veröffentlichungstag der Anmeldung: **13.04.83 Patentblatt 83/15**

(45) Bekanntmachung des Hinweises auf die Patenterteilung: **15.05.85 Patentblatt 85/20**

(84) Benannte Vertragsstaaten: **AT BE CH DE FR GB IT LI LU NL**

(56) Entgegenhaltungen:
**CH-A- 562 191**
**CH-A- 570 354**
**JOURNAL OF THE CHEMICAL SOCIETY, Perkin transactions I, 1979, London T.KATO et al. "Studies on Keten and its derivatives. Part 89. Ethyl 4- Substituted Acetoacetates: Synthesis and Reaction with Diketen" Seiten 529 bis 532**

(73) Patentinhaber: **LONZA AG**
**Gampel/Wallis (CH)**

(72) Erfinder: **Greth, Erich, Dr. Dipl.-Ing.Chem. ETH**
**Hirschweg 16**
**Visp (CH)**

(74) Vertreter: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8**
**D-8000 München 40 (DE)**

**EP 0 076 379 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 4-Alkoxyacetessigestern.

Es ist bekannt, 4-Ethoxyacetessigester durch Reaktion von Bromessigester und Ethoxyessigester mit Zink (J. Amer. Chem. Soc., 68, 1946, 2392) oder durch Reaktion von Ethoxyessigester mit Essigester in Gegenwart von Natrium (Chem. Abstr., 43, 1949, 2625e) herzustellen. Es ist auch bekannt, den 4-Methoxyacetessigester durch Kondensation von Methoxyacethylchlorid mit Malonsäureäthyltertiärbutylester mit nachfolgender Verseifung und Decarboxylierung herzustellen (J. Amer. Chem. Soc., 70, 1948, S. 500). Die nach diesem Verfahren erzielten Ausbeuten liegen bei 11, 21 bzw. 40 %.

Versuche, 4-Ethoxyacetessigester aus 4-Chloracetessigester mit äquimolaren Mengen Na-Alkoholaten in Alkohol herzustellen, schlugen zunächst fehl. Anstelle des erwarteten 4-Ethoxyacetessigesters wurde nämlich Succinylbernsteinsäureester erhalten (Bull. Soc. Chim. France, 4. Serie, 29, 1921, S. 402-406).

Nach Schweizer Patent 562 191 ist es dann doch gelungen, 4-Alkoxyacetessigester aus den 4-Halogenacetessigestern mit Alkalialkoholaten herzustellen, wenn man in einem Gemisch von Alkohol und aprotischem Lösungsmittel mit hoher Dielektrizitätskonstante, vorzugsweise Dimethylsulfoxid, bei Temperaturen von 15 bis 30 °C arbeitet. Der Nachteil dieses Verfahrens besteht darin, dass Reaktionszeiten von 24 bis 72 Stunden benötigt werden und die Reaktion in einer grossen Menge des Lösungsmittelgemisches durchgeführt werden muss. Ausserdem erschwert Dimethylsulfoxid die Isolation der 4-Alkoxyacetessigester.

Nach T. Kato (J. Chem. Soc., Perkin I, 529 (1979)) ist es bekannt, 4-Ethoxyacetessigester aus 4-Bromacetessigester herzustellen. Dabei wird 1 Äquivalent 4-Bromacetessigester mit 2,2 Äquivalenten Natriumethylat umgesetzt. Als Lösungsmittel dienen grosse Mengen Ethanol, die Ausbeuten sind mit 47 % sehr bescheiden.

Der Erfindung liegt die Aufgabe zugrunde, ein Verfahren zur Herstellung der bisher schwer zugänglichen 4-Alkoxyacetessigester zu finden, das hohe Ausbeuten bei einfacher Durchführung und kurzer Reaktionszeit gestattet.

Erfindungsgemäss wird dies dadurch erreicht, dass man 4-Chlor- bzw. 4-Bromacetessigester mit einer bei erhöhter Temperatur hochkonzentrierten Lösung von Alkalialkoholat in Alkohol, wobei pro Mol 4-Halogenacetessigester mindestens 2,8 Mol Alkoholat verwendet wird, bei erhöhter Temperatur umsetzt.

Das Alkalialkoholat wird in Konzentrationen von 10 Gew.-% bis gegen 40 Gew.-% eingesetzt, vorzugsweise 20-35 Gew.-%.

Diese Angaben bedeuten Gew.-% in der Lösung bei Beginn der Reaktion.

Bei den niederen Alkoholen (Methanol/Ethanol) handelt es sich um richtige Lösungen, bei höheren Alkoholen können auch Lösungen eingesetzt werden, die noch ungelöstes Alkoholat enthalten (Suspension).

Der Konzentrationsbereich ist nach unten durch die abfallende Ausbeute begrenzt und nach oben im wesentlichen durch die Rührbarkeit des Reaktionsgemisches. Bei der Reaktion entsteht Alkalihalogenid, welches als Feststoff ausfällt ; bei zu konzentrierter Arbeitsweise führt das ausgefallene Alkalihalogenid zu unrührbaren Reaktionsgemischen.

Solche konzentrierte Alkoholatlösungen können so erhalten werden, dass man das entsprechende Alkalimetall vorlegt und den Alkohol langsam zudosiert und endlich noch auf eine Temperatur von 50-120 °C erhitzt, bis alles Metall gelöst ist. Solche konzentrierte Alkoholatlösungen können aber auch hergestellt werden durch Auflösen oder Suspendieren von festem Alkalialkoholat im entsprechenden Alkohol.

Die Temperatur zum Auflösen von Alkalimetall oder Alkalialkoholat beträgt ca. 50-120 °C. Bei tieferer Temperatur werden keine für die Reaktion ausreichenden Konzentrationen erreicht, bei höheren besteht die Gefahr der Zersetzung. Bei Methanol und Ethanol wird vorzugsweise bei Rückflusstemperatur gearbeitet.

Vorzugsweise wird in diese gesättigte Alkalialkoholatlösung der 4-Halogenacetessigester eindosiert.

Als Reaktionstemperatur wählt man vorzugweise die gleiche Temperatur, bei der man die Herstellung der Alkalialkoholatlösung betrieben hat, doch ist dies nicht zwingend.

Nach unten ist die Reaktionstemperatur begrenzt durch steigende Reaktionszeiten sowie durch die sinkende Löslichkeit der Alkalialkoholate, nach oben durch mögliche Zersetzung von Edukten oder Produkten. Bei Reaktionen in Methanol oder Ethanol ist Rückflusstemperatur optimal.

Die Reaktionszeit beträgt zweckmässig 15 Minuten bis mehrere Stunden (in Abhängigkeit von der Reaktionstemperatur), vorzugsweise 30 Minuten bis 1 Stunde.

Das Alkalialkoholat wird vorteilhaft in Mengen von über 2,8 Mol, vorzugsweise 3 bis 4 Mol, pro Mol 4-Halogenacetessigester angewendet.

Als Alkalialkoholat kommen insbesondere die Natrium- und Kaliumsalze zur Anwendung. Als Alkoholkomponente können alle, die sich von aliphatischen Alkoholen ableiten, zweckmässig solche, die 1 bis 4 C-Atome im Molekül aufweisen, die geradkettig oder verzweigt sein können, verwendet werden. Solche Alkohole sind beispielsweise Methanol, Aethanol, Butanol, sek. Butanol, Propanol, Isopropanol.

Von den 4-Chlor- bzw. 4-Bromacetessigestern kommen insbesondere die 4-Chlorderivate zur

Anwendung. Dabei können auch solche eingesetzt werden, die in 2-Stellung einen Substituenten tragen. Somit gelingt es nach dem Verfahren der Erfindung, 4-Alkoxyacetessigester der allgemeinen Formel

$$R - O - CH_2 - \underset{\underset{O}{\parallel}}{C} - \overset{\overset{R_1}{|}}{CH} - COOR_2$$

in welcher R und $R_2$ Alkylgruppen und $R_1$ = H oder Alkyl ist, herzustellen.

Die Substituenten R, $R_1$ und $R_2$ unterliegen keinen zahlenmässigen Beschränkungen der C-Atome. Beispielsweise können sie je 1 bis 10, insbesondere 1 bis 4 Kohlenstoffatome aufweisen. Es können alle Alkylgruppen, ob geradkettig oder verzweigt, Anwendung finden. Auch ist es möglich, substituierte Alkylgruppen zu verwenden (z. B. Methoxy, Alkyl, z. B. $C_1$-$C_{10}$-Alkyl, Aryl, z. B. Phenyl). Wesentliches Merkmal der Substituenten muß sein, daß sie in stark basischem Medium nicht reagieren.

Besonders geeignet ist das Verfahren der Erfindung zur Herstellung von 4-Alkoxyacetessigestern, bei denen die 4-Alkoxygruppe und die Alkoholgruppe des Esters gleich sind. Wendet man dieses Verfahren auf die Herstellung von 4-Alkoxyacetessigester an, bei denen die 4-Alkoxygruppe verschieden von der Alkoholgruppe des Esters ist, erhält man durch Umesterung Estergemische.

Aus dem nach der Reaktion vorliegenden Reaktionsgemisch wird der 4-Alkoxyacetessigester vorzugsweise nach folgender Methode gewonnen.

Zum Reaktionsgemisch wird unter Rühren eine Mineralsäure (Salzsäuregas, Schwefelsäure) so zudosiert, dass am Ende ein pH-Wert der Neutralisation von 5 bis 7 erzielt wird. Die neutralisierte Reaktionslösung lässt sich weiter auf verschiedenen Wegen zum gewünschten 4-Alkoxyacetessigester aufarbeiten. Zum Beispiel wird die die Reaktionslösung in einem Rotationsverdampfer vom Alkohol befreit, zweckmässig bei 20 Torr Vakuum, und der Rückstand im Wasser aufgenommen. Diese wässrige Phase wird mit einem Lösungsmittel, z. B. mit Essigester, extrahiert und das Lösungsmittel abdestilliert.

## Beispiel 1

Ein Sulfierkolben wurde mit Stickstoff gespült und mit 90 g Natrium beschickt und tropfenweise 530 g Methanol zugesetzt. Nach beendeter Zugabe wurde im Oelbad bis zum Rückfluss erhitzt, bis unter Rühren alles Natrium gelöst war (3 bis 4 Stunden). Nun wurde das Oelbad entfernt und unter heftigem Rühren 201, 30 g 4-Chloracetessigmethylester zulaufen gelassen (ca. 6 Minuten). Nach beendeter Zugabe wurde das Oelbad wieder zurückgebracht, 30 Minuten am Rückfluss gekocht, und bei Siedetemperatur mit Salzsäuregas und mit Hilfe eines pH-Meters und einer komb. Glaselektrode mit Salzsäuregas bis pH 6,65 neutralisiert. Diese Reaktionslösung wurde am Rotationsverdampfer bei 20 Torr Endvakuum/60 bis 70 °C Badetemperatur vom Methanol befreit, der Rückstand in 650 ml Wasser aufgenommen und 5 mal mit Essigester (1 × 200 ml, 4 × 100 ml) extrahiert und der Essigester über eine kurze Destillationskolonne abdestilliert.

Es resultierten 203,70 g Rohprodukt.

Dieser rohe Ester wurde bei 80 °C/8 Torr über eine 60 cm Vigreuxkolonne mit Vakuummantel destilliert.

Es resultierten 158,49 g destillierter 4-Methoxyacetessigmethylester als farblose Flüssigkeit, entsprechend einer Ausbeute von 80,57 %. Der Gehalt lag bei 97,8 %.

## Beispiele 2 und 3

Wie in Beispiel 1 wurden weitere Versuche durchgeführt. Als Ausgangsverbindungen wurden 4-Chloracetessigsäureoctylester und als Alkoholate einmal Natriumäthylat und Natriumoctanat eingesetzt.

Die erzielten Resultate waren in Beispiel 2 80,8 %, in Beispiel 3 79,2 %, bezogen auf eingesetzten Chlorester.

## Beispiel 4

(Vergleichsbeispiel, ist nicht Gegenstand der Erfindung)

15,56 g 4-Chloracetessigsäuremethylester (0,105 Mol) wurden in 200 ml Methanol mit 5,51 g Natrium (0,240 Mol, d. h. 2,28 Mol pro Mol 4-Chloracetessigmethylester) bei 20 °C während 48 Stunden reagieren gelassen. Man isolierte ein schwarzes Oel, in dem sich analytisch 5,31 g 4-Methoxyacetessigester nachweisen lassen, entsprechend 35,2 % Ausbeute bez. einges. 4-chloracetessigmethylester.

Daneben erhält man 6,15 g nicht-flüchtige Nebenprodukte (Harz). Auf 1 Teil Produkt bilden sich 1,16 Teile Harz.

## Beispiel 5

(Vergleichsbeispiel, ist nicht Gegenstand der Erfindung)

7,24 g 4-Chloracetessigmethylester (0,049 Mol) wurden mit einer Lösung von 5,75 g (0,25 Mol, 5,1 Mol pro Mol 4-Chloracetessigmethylester) in 200 ml Methanol während einer Stunde bei Rückflusstemperatur umgesetzt :

Im Reaktionsprodukt konnte man 362 g 4-Methoxyacetessigmethylester nachweisen, entspr. 51,7 % Ausbeute (bez. einges. 4-Chloracetessigester) daneben erhielt man 2,63 g nicht-flüchtiges Harz. Auf 1 Teil Produkt erhält man 0,73 Teile nichtflüchtiges Nebenprodukt.

### Beispiel 6

14,75 g 4-Chloracetessigmethylester (0,10 Mol) wurden in einer Lösung von 23,06 g Natrium (1 Mol, 10-facher Ueberschuss) in 200 ml Methanol während 1 Stunde rückflussiert.

Im Reaktionsprodukt werden 13,26 g 4-Methoxyacetessigmethylester nachgewiesen, was 92,7 % Ausbeute entspricht. Daneben bestimmt man 0,44 g nicht-flüchtige Nebenprodukte (Harze). Auf 1 Teil Produkt erhält man lediglich 0,033 Teile Harze.

### Beispiel 7

30,70 g 4-Chloracetessigmethylester (0,208 Mol) wurden mit einer Lösung von 22,95 g Natrium (0,999 Mol, 4,8-facher Ueberschuss) in 200 ml Methanol 1 Std. rückflussiert.

Im Reaktionsprodukt werden 25,97 g 4-Methoxyacetessigester nachgewiesen, entspr. 87,2 % Ausbeute bez. auf 4-Chloracetessigmethylester. Daneben erhält man 1,76 g Harz. Auf 1 Teil Produkt erhält man 0,068 Teile Harz.

### Beispiel 8

Wie im Beispiel 1 wurden 69,0 g metallisches Natrium in 500 ml Ethanol bei Rückflusstemperatur gelöst. In diese Lösung lässt man 151,63 g 4-Chloracetessigsäureethylester einlaufen ; die Zugabe dauert 15 Minuten ; während der Zugabe wurde auf Rückflusstemperatur gehalten. Nach beendeter Zugabe wurde weitere 15 Minuten rückflussiert, dann wurde mit konzentrierter Schwefelsäure auf einen pH-Wert von ca. 6 neutralisiert und wie in Beispiel 1 aufgearbeitet. Man erhält 162,47 g rohen 4-Ethoxyacetessigester ; die Destillation gibt 147,05 g 4-Ethoxyacetessigester mit einem Gehalt von 96,8 % (Gaschromatographie), entsprechend 142,35 g 100 %-ig, entsprechend 88,7 % Ausbeute, bez. auf 4-Chloracetessigethylester.

Als Destillationsrückstand bleiben 9,80 g nicht-flüchtiger Rückstand, das sind 0,069 Teile auf 1 Teil 4-Ethoxyacetessigsäureethylester 100 %-ig.

## Patentansprüche

1. Verfahren zur Herstellung von 4-Alkoxyacetessigsäureestern der allgemeinen Formel

$$R - O - CH_2 - \underset{\underset{O}{\|}}{C} - \overset{R_1}{\overset{|}{CH}} - COOR_2$$

in welcher R und $R_2$ Alkylgruppen und $R_1$ = H oder Alkyl ist, aus 4-Chlor- bzw. 4-Bromacetessigestern, dadurch gekennzeichnet, daß man 4-Chlor- bzw. 4-Bromacetessigester mit einer 10 bis 40 Gew.-prozentigen Lösung von Alkalialkoholat in Alkohol, wobei pro Mol 4-Chlor- bzw. 4-Bromacetessigester mindestens 2,8 Mol Alkoholat angewendet werden, bei Temperaturen von 50 bis 120 °C umsetzt.

2. Verfahren gemäss Patentanspruch 1, dadurch gekennzeichnet, dass man pro Mol 4-Chlor- bzw. 4-Bromacetessigester 3 bis 4 Mol Alkalialkoholat anwendet.

## Claims

1. Process for the production of 4-alkoxyaceto acetic acid ester of the general formula

$$R - O - CH_2 - \underset{\underset{O}{\|}}{C} - \overset{R_1}{\overset{|}{CH}} - COOR_2$$

in which R and $R_2$ are alkyl groups and $R_1$ is H or alkyl, from 4-chloro- or 4-bromoaceto acetic acid ester is reacted at temperatures of 50 to 120 °C with a 10 to 40 percent by weight solution of alkali alcoholate in

alcohol using at least 2.8 moles of alcoholate per mole of 4-chloro- or 4-bromaceto acetic acid ester.

2. Process according to patent claim 1, characterized in that per mole of 4-chloro- or 4-bromoaceto acetic acid ester 3 to 4 moles of alkali alcoholate are used.

**Revendications**

1. Procédé pour la préparation d'esters d'acides 4-alcoxyacétylacétiques de formule générale

$$R - O - CH_2 - \underset{\underset{O}{\Vert}}{C} - \overset{\overset{R_1}{|}}{CH} - COOR_2$$

dans laquelle R et $R_2$ sont des groupes alcoyle et $R_1$ = H ou est alcoyle, à partir d'esters d'acides 4-chloro- ou respectivement 4-bromoacétylacétiques, caractérisé en ce qu'on fait réagir à des températures de 50 à 120 °C, des esters d'acides 4-chloro- ou respectivement 4-bromoacétylacétiques avec une solution à 10 à 40 pour cent en poids d'alcoolate alcalin dans l'alcool, en utilisant par mole d'ester d'acide 4-chloro- ou respectivement 4-bromoacétylacétique au moins 2,8 moles d'alcoolate.

2. Procédé selon la revendication 1, caractérisé en ce que l'on utilise, par mole d'ester d'acide 4-chloro- ou respectivement 4-bromoacétylacétique, 3 à 4 moles d'alcoolate alcalin.